# EUROPÄISCHE PATENTSCHRIFT

(11) **EP 1 654 946 B1**
(45) Veröffentlichungstag und Bekanntmachung des Hinweises auf die Patenterteilung: **04.01.2012**
(21) Anmeldenummer: 05024178.5
(22) Anmeldetag: 07.11.2005
(51) Int. Cl.: A43B 17/14, A43D 39/00, A61F 5/14, A43B 7/22, A43D 1/02

(54) **Einlage für einen Schuh und Verfahren zur Herstellung einer Einlage für einen Schuh**
Orthotic for a shoe and method of manufacturing an orthotic for a shoe
Semelle intérieure pour chaussure et procédé de fabrication d'une semelle intérieure

(30) Priorität: 05.11.2004 DE 102004053930; 05.11.2004 DE 202004017280 U
(43) Veröffentlichungstag der Anmeldung: 10.05.2006
(73) Patentinhaber: Klapdor, Axel, 41749 Viersen (DE)
(72) Erfinder: Klapdor, Axel, 41749 Viersen (DE)
(74) Vertreter: DR. STARK & PARTNER

(56) Entgegenhaltungen:
- EP-A- 0 508 963
- EP-A- 1 584 465
- US-A- 4 454 618
- US-A- 5 054 148
- US-A- 6 006 412

## Beschreibung

Die Erfindung betrifft ein Verfahren zur Herstellung einer Einlage für einen Schuh, die eine während der Benutzung zumindest mit der Fußsohle des Benutzers der Einlage in Kontakt stehende Oberseite und eine während der Benutzung zumindest mit der unteren Innenfläche des Schuhs in Kontakt stehende Unterseite aufweist, und die mit einer auf den Benutzer und insbesondere auch auf den Schuh, abgestimmten Form versehen ist.

Aus der Praxis sind derartige Einlagen bekannt, die entweder zur Erzielung eines "orthopädisch" korrekten Fußbettes im Schuh dienen oder aber eine Fehlhaltung des Benutzers oder eine Fehlbildung des entsprechenden Fußbettes des Benutzers kompensieren sollen.

Üblicherweise werden solche Einlagen aus mehreren dünnen Lagen unterschiedlicher Härte und/oder Zusammensetzung miteinander verklebt und in Form gepresst, wobei insbesondere für die Erhöhung im Mittelfuß noch ein relativ weiches Füllmaterial zwischen die verschiedenen Schichten eingebracht wird.

Nachteilig hierbei ist, dass eine sehr dünne und relativ harte Einlage resultiert, die zudem konstruktionsbedingt keine insbesondere zur seitlichen Führung des mittleren Innenfußes oder der Ferse dienende stark erhöhten Bereiche in stabiler Ausgestaltung aufweisen kann.

Sofern nach erfolgter Herstellung der Einlage, z. B. aufgrund von Tragebeschwerden des Benutzers, eine Anpassung der Einlage erforderlich wird, ist dies hinsichtlich des topografischen Verlaufs prinzipiell nicht möglich, da allenfalls die Erhöhung im Mittelfuß kann durch Materialentnahme etwas reduziert werden. Hierfür ist jedoch eine Ablösung der oberen Schicht erforderlich, wodurch diese in der Regel in Mitleidenschaft gezogen wird, so dass häufig zumindest die Deckschicht komplett ersetzt werden muss oder aber die Anfertigung einer neuen Einlage erforderlich wird.

Aufgabe der Erfindung ist es, die vorgenannten Nachteile zu vermeiden und ein Verfahren zur Herstellung einer Einlage für einen Schuh anzugeben, mit der zum einen die Herstellung einer solchen Einlage schneller und einfacher erfolgen kann und zum anderen auch spätere Anpassungen problemlos möglich sind, ohne dass hierbei zumindest die Oberseite der Einlage in Mitleidenschaft gezogen wird.

Diese Aufgabe wird gelöst durch ein gattungsgemäßes Verfahren, wobei die Einlage personalisiert und einstückig aus einem Vollmaterial, insbesondere durch Materialabtrag, wie z. B. Fräsen, hergestellt wird und zumindest oberseitig mit einer auf den konkreten Benutzer und insbesondere auch auf den Schuh abgestimmten Topografie versehen wird.

Hierdurch ist eine besonders einfache Herstellung der Einlage möglich, da diese ohne einzelnes Vorkonfektionieren und Zusammenfügen verschiedener Bestandteile erfolgt.

*Dabei weist das Vollmaterial einen dreischichtigen Aufbau auf, der eine unterseitige Schicht mit höherer Härte, einen mittleren Bereich geringerer Härte und einen oberen Bereich mit wieder höherer Härte beinhaltet.*

Vorzugsweise kann das Vollmaterial ein Thermoplast, insbesondere EVA (Ethylenvinylacetat) sein, und das Vollmaterial *weist* einen mehrschichtigen Aufbau mit Schichten unterschiedlicher Härte auf, so dass sowohl eine einfache Herstellung des Vollmaterials möglich ist und durch den mehrschichtigen Aufbau in Abhängigkeit von der üblicherweise zu erwartenden Topografie eine gezielte Abstimmung der bei der fertiggestellten Einlage verbleibenden Bereiche hinsichtlich ihrer Härte und Wirkung erfolgen kann.

Erfindungsgemäß können die Schichten eine unterschiedliche Struktur und/oder verschiedene Farben aufweisen, so dass die jeweiligen Materialeigenschaften auch an der fertiggestellten Einlage erkennbar sind. Hierdurch können auch ästhetische Effekte erzielt werden. Zu diesem Zweck kann die Färbung auch unabhängig von der jeweiligen Härte und Materialstruktur ausgebildet sein.

Vorteilhafterweise kann sowohl die Oberseite als auch die Unterseite mit einer zumindest weitgehend standardisierten Topographie versehen werden, so dass eine Art Rohlingfertigung für verschiedene Schuhtypen, Schuhgrößen oder Fehlstellungen/Fehlhaltungen möglich ist, die dann zur Anpassung an den konkreten Fall nur noch geringfügig nachgearbeitet werden müssen.

Hierzu kann bei einer bevorzugten Variante der Erfindung in die standardisierte Topografie der Unterseite zur Anpassung der oberseitigen Topografie der Einlage während der Benutzung an den Benutzer und insbesondere auch an den Schuh zusätzlich unterseitig wenigstens ein durch Materialabtrag, insbesondere Fräsen hergestellter Freibereich eingebracht werden, so dass die mit einer hohen Oberflächengüte versehene Oberseite des Rohlings keine visuellen erkennbaren Nacharbeitungsspuren aufweist.

Vorteilhafterweise kann der Materialabtrag, bei der Herstellung des Rohlings und/oder bei der Anpassung an den konkreten Benutzer, konkreter computergesteuert erfolgen, dass eine einfache und sehr genaue Fertigung möglich ist.

Erfindungsgemäß kann der Materialabtrag entsprechend einer 3D-Messung des Fußes und/oder einer Ganganalyse erfolgen, so dass eine hohe Passgenauigkeit erzielt wird und eine sehr gute Anpassung an die konkreten Erfordernisse unter Berücksichtigung der Anatomie und der Bewegungsabläufe des Benutzers möglich ist.

Hierbei kann der Materialabtrag auch entsprechend einer beabsichtigten Kompensation einer diagnostizierten Fehlstellung erfolgen.

Die Erfindung betrifft weiterhin auch eine Einlage für einen Schuh, die eine während der Benutzung zumindest mit der Fußsohle des Benutzers der Einlage in Kontakt stehende Oberseite und eine während der Benutzung zumindest mit der unteren Innenfläche des Schuhs in Kontakt stehende Unterseite aufweist, und die mit einer auf den Benutzer und insbesondere auch auf den Schuh, abgestimmten Form versehen ist.

Die Nachtteile bereits vorbekannter Einlagen wurden eingangs bereits diskutiert.

Es ist insoweit auch Aufgabe der Erfindung, eine Einlage für einen Schuh anzugeben, deren Herstellung zum einen schneller und einfacher erfolgen kann und bei der zum anderen auch spätere Anpassungen problemlos möglich sind, ohne dass hierbei zumindest die Oberseite der Einlage in Mitleidenschaft gezogen wird.

Diese Aufgabe wird gelöst durch eine gattungsgemäße Einlage, wobei die Einlage personalisiert und einstückig aus einem Vollmaterial, insbesondere durch Materialabtrag, wie z. B. Fräsen, hergestellt ist und zumindest oberseitig eine auf den konkreten Benutzer und insbesondere auch auf den Schuh abgestimmte Topografie aufweist.

*Dabei weist das Vollmaterial einen dreischichtigen Aufbau auf, der eine unterseitige Schicht mit höherer Härte, einen mittleren Bereich geringerer Härte und einen oberen Bereich mit wieder höherer Härte beinhaltet.*

Hierdurch ist aufgrund des speziellen Aufbaus der Einlage diese einfach herstellbar, da kein einzelnes Vorkonfektionieren und Zusammenfügen verschiedener Bestandteile bei der Herstellung erforderlich ist.

Vorzugsweise kann das Vollmaterial ein Thermoplast, insbesondere EVA (Ethylenvinylacetat) sein, und das Vollmaterial *weist* einen mehrschichtigen Aufbau mit Schichten unterschiedlicher Härte auf , so dass sowohl eine einfache Herstellung des Vollmaterials möglich ist und durch den mehrschichtigen Aufbau in Abhängigkeit von der üblicherweise zu erwartenden Topografie eine gezielte Abstimmung der bei der fertiggestellten Einlage verbleibenden Bereiche hinsichtlich ihrer Härte und Wirkung erfolgen kann.

Erfindungsgemäß können die Schichten eine unterschiedliche Struktur und/oder verschiedene Farben aufweisen, so dass die jeweiligen Materialeigenschaften auch an der fertiggestellten Einlage erkennbar sind. Hierdurch können auch ästhetische Effekte erzielt werden. Zu diesem Zweck kann die Färbung auch unabhängig von der jeweiligen Härte und Materialstruktur ausgebildet sein.

Vorteilhafterweise kann sowohl die Oberseite als auch die Unterseite mit einer zumindest weitgehend standardisierten Topographie versehen werden, so dass eine Art Rohlingfertigung für verschiedene Schuhtypen, Schuhgrößen oder Fehlstellungen/Fehlhaltungen möglich ist, die dann zur Anpassung an den konkreten Fall nur noch geringfügig nachgearbeitet werden müssen.

Hierzu kann bei einer bevorzugten Variante der Erfindung die Unterseite in ihrer zumindest weitgehend standardisierten Topografie zur Anpassung der oberseitigen Topografie während der Benutzung an den Benutzer und insbesondere auch an den Schuh zusätzlich unterseitig wenigstens einen durch Materialabtrag hergestellten, insbesondere ausgefrästen, Freibereich aufweisen, so dass die mit einer hohen Oberflächengüte versehene Oberseite des Rohlings nach erfolgter Anpassung der Einlage keine visuellen erkennbaren Nacharbeitungsspuren aufweist.

Erfindungsgemäß kann der bei der Herstellung der Einlage erfolgte Materialabtrag entsprechend einer 3D-Messung des Fußes und/oder einer Ganganalyse erfolgt sein, so dass eine hohe Passgenauigkeit erzielt wird und eine sehr gute Anpassung an die konkreten Erfordernisse unter Berücksichtigung der Anatomie und der Bewegungsabläufe des Benutzers möglich ist.

Hierbei kann der Materialabtrag auch entsprechend einer beabsichtigten Kompensation einer diagnostizierten Fehlstellung erfolgt sein.

Im Folgenden wird ein in der Zeichnung dargestelltes Ausführungsbeispiel der Erfindung erläutert. Es zeigen:
- Fig. 1: eine perspektivische Ansicht schräg von oben auf eine erfindungsgemäße Einlage,
- Fig. 2: die Schnittdarstellung X-X des Gegenstandes von Fig.1,
- Fig. 3: den Schnitt Y-Y des Gegenstandes nach Fig. 1 und

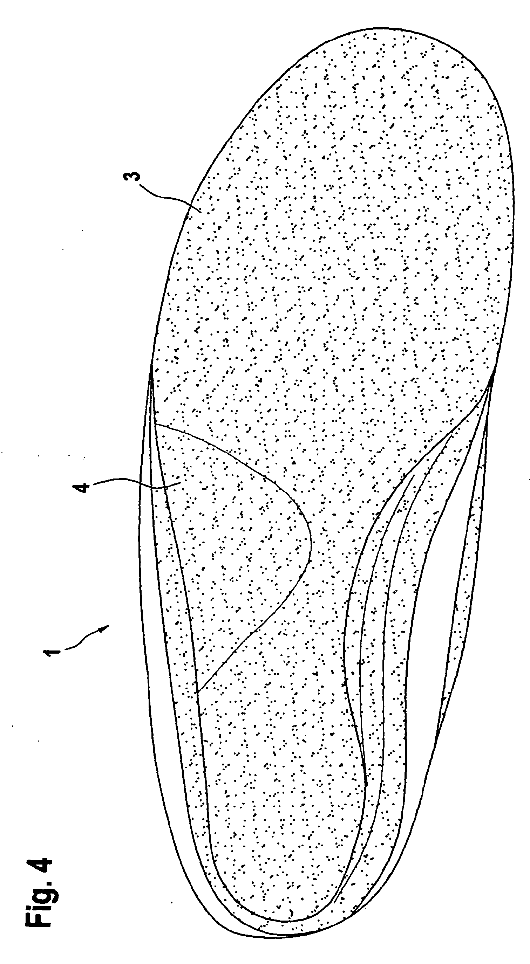

- Fig. 4: eine Unteransicht des Gegenstandes nach Fig. 1.

In allen Figuren werden für gleiche bzw. gleichartige Bauteile übereinstimmende Bezugszeichen verwendet.

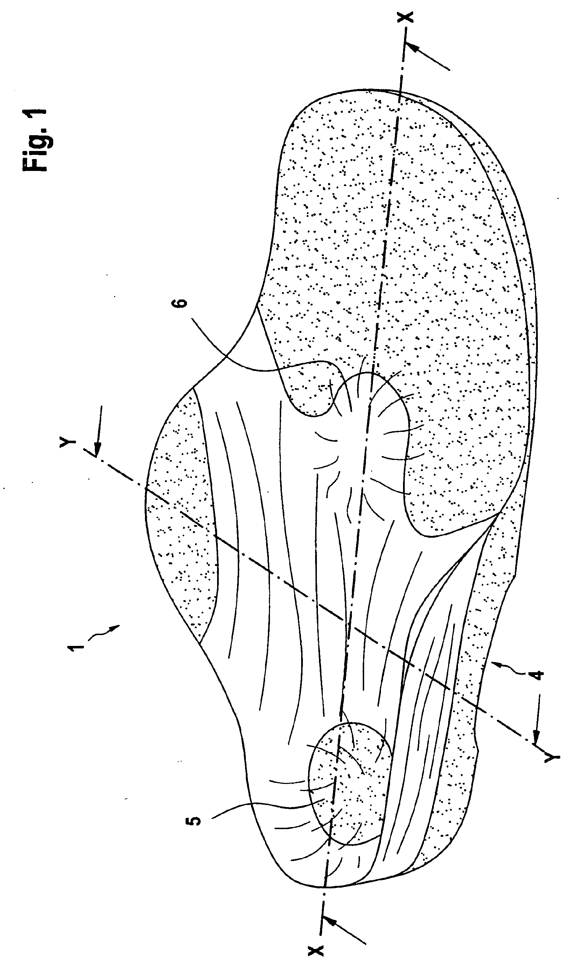

Fig. 1 zeigt eine Einlage 1 für einen in der Zeichnung nicht dargestellten Schuh, die sowohl eine Oberseite 2 als auch eine Unterseite 3 aufweist. Während der Benutzung im Schuh steht die Unterseite 3 zumindest mit der unteren Innenfläche des Schuhs, gegebenenfalls aber auch mit seitlichen Innenflächen des Schuhs in Kontakt, wohingegen die Fußsohle des Benutzers auf der Oberseite 2 der Einlage 1 aufliegt.

Die Einlage 1 weist dabei eine äußere Form auf, die auf den Benutzer und insbesondere auch auf den Schuh, in dem sie eingesetzt werden soll, abgestimmt ist. Dies ist allein schon sinnvoll, damit die Einlage 1 später in den Schuh passgenau eingesetzt werden kann.

Die Einlage 1 ist aus einem Vollmaterial, wie z. B. einem geschäumten Thermoplast (EVA oder andere) hergestellt und weist zumindest oberseitig eine auf den Benutzer und insbesondere auch auf den Schuh abgestimmte Topografie auf. Das bedeutet, dass der Höhenverlauf der zumindest der Oberseite 2 der Einlage 1 auf den Benutzer und den Schuh abgestimmt ist.

Insoweit kann bei Einlagen 1, die in Turnschuhen eingesetzt werden sollen, die Unterseite 3 flach und eben ausgebildet sein, um die Einlage 1 im Austausch gegen das herausnehmbare Fußbett des Turnschuhs zu verwenden.

Sofern die Einlage 1 in Business-Schuhen oder aber anderen Schuhen mit Absatz verwendet werden soll, kann auch die Unterseite eine entsprechend auf den Schuh abgestimmte Topografie aufweisen.

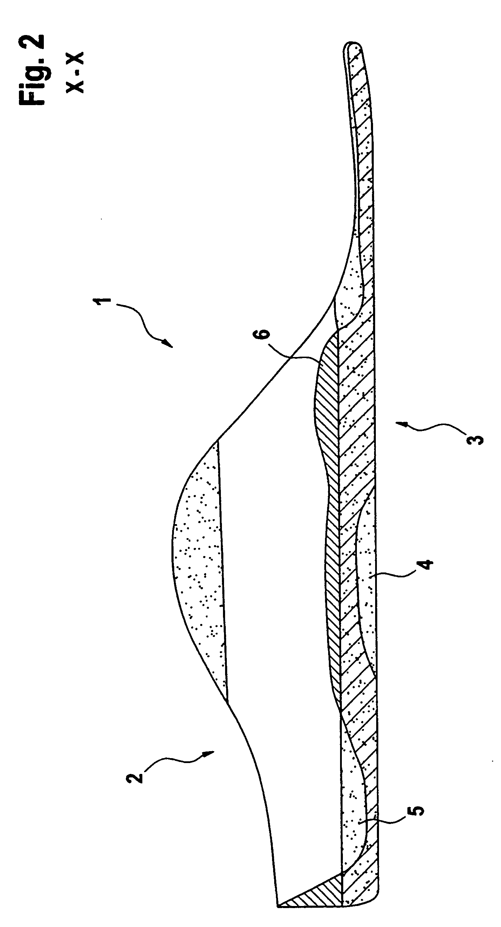

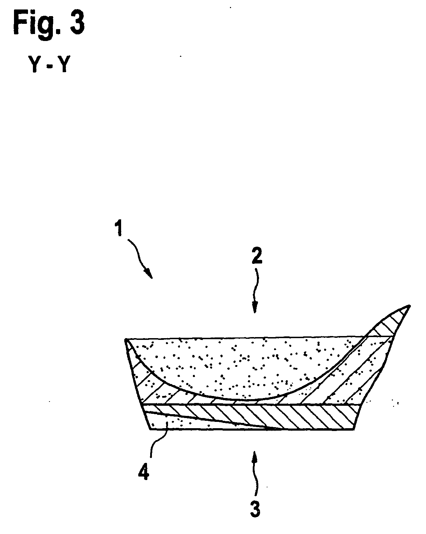

Das in den Zeichnungen dargestellte Ausführungsbeispiel einer Einlage 1 weist, wie insbesondere aus den Fig. 2 und 3 ersichtlich, einen dreischichtigen Aufbau auf, der eine unterseitige Schicht mit höherer Härte (in der Zeichnung gepunktet dargestellt), einen mittleren Bereich geringerer Härte und einen oberen Bereich mit wieder höherer Härte (in der Zeichnung gepunktet dargestellt) beinhaltet.

Sofern es sich bei der Einlage 1 um eine Maßanfertigung handelt, kann diese bei der Herstellung direkt passgenau unter Berücksichtigung aller Anforderungen auf den jeweiligen Besitzer und den damit zu kombinierenden Schuh abgestimmt werden.

Alternativ kann aber auch die Oberseite 2 und die Unterseite 3 eine weitgehend standardisierte Topografie für bestimmte Anwendungszwecke aufweisen und zur Anpassung der Passform der Einlage 1 kann die Unterseite 3 der Einlage 1 zusätzlich zu ihrer standardisierten Topografie wenigstens einen Freibereich 4 aufweisen, der durch Materialabtrag, insbesondere Fräsen, hergestellt wird.

Zwar bleibt im unbelasteten Zustand aufgrund der gewissen Steifigkeit der Einlage 1 die oberseitige weitgehend standardisierte Topografie erhalten, im belasteten Zustand verformt sich die Einlage 1 durch die zumindest den einen unterseitigen Freibereich 4, so dass durch eine Änderung der unterseitigen Topografie im Benutzungszustand eine Änderung der oberseitigen Topografie resultiert.

Zur Visualisierung und auch für ästhetische Effekte können die verschieden harten Schichten ein unterschiedliches Aussehen, insbesondere unterschiedliche Farben aufweisen, wohingegen für ein schlichtes und einheitliches Erscheinungsbild die unterschiedlich harten Schichten ein einheitliches Aussehen haben können.

Insbesondere ist es auch möglich, dass eine Schicht bestimmter Härte in verschiedene weitere Unterschichten gleicher Härte, aber unterschiedlicher Erscheinungsbilder, insbesondere unterschiedlicher Farben, aufgeteilt ist, um somit ästhetische Effekte zu erzielen und/oder die Topografie zu visualisieren. Insoweit können auch zwischen wiederum gleichfarbigen Schichten jeweils kontrastfarbene Zwischenschichten vorgesehen sein, so dass ein Topografie-Linienbild in der Art von Höhenlinien resultiert.

Die in der Zeichnung dargestellte Einlage 1 weist vertiefte und erhöhte Bereiche auf, von denen insbesondere eine Vertiefung 5 im Bereich der Ferse und eine Erhöhung 6 im Bereich des Mittelfußes deutlich zu erkennen sind. Des Weiteren sind im Bereich des Mittelfußes seitliche Erhöhungen 6 vorgesehen, von denen die Erhöhung an der Fußaußenseite sich nur im Bereich einer Schicht befindet und die Erhöhung an der Fußinnenseite sich über zwei Schichten erstreckt.

## Patentansprüche

1. Verfahren zur Herstellung einer Einlage (1) für einen Schuh, die eine während der Benutzung zumindest mit der Fußsohle des Benutzers der Einlage (1) in Kontakt stehende Oberseite (2) und eine während der Benutzung zumindest mit der unteren Innenfläche des Schuhs in Kontakt stehende Unterseite (3) aufweist, und die mit einer auf den Benutzer und insbesondere auch auf den Schuh, abgestimmten Form versehen ist, ***wobei*** die Einlage (1) personalisiert und einstückig aus einem Vollmaterial, durch Materialabtrag, wie z. B. Fräsen, hergestellt wird und zumindest oberseitig mit einer auf den konkreten Benutzer und insbesondere auch auf den Schuh abgestimmten Topografie versehen wird, **dadurch gekennzeichnet, dass** *das Vollmaterial einen dreischichtigen Aufbau aufweist, der eine unterseitige Schicht mit höherer Härte, einen mittleren Bereich geringerer Härte und einen oberen Bereich mit wieder höherer Härte beinhaltet.*

2. Verfahren nach Anspruch 1, **dadurch gekennzeichnet, dass** das Vollmaterial ein Thermoplast, insbesondere EVA (Ethylenvinylacetat) ist.

3. Verfahren nach einem der vorhergehenden Ansprüche, **dadurch gekennzeichnet, dass** sowohl die Oberseite (2) als auch die Unterseite (3) mit einer zumindest weitgehend standardisierten Topographie versehen werden.

4. Verfahren nach Anspruch 3, **dadurch gekennzeichnet, dass** in die standardisierte Topografie der Unterseite (3) zur Anpassung der oberseitigen Topografie der Einlage 81) während der Benutzung an den Benutzer und insbesondere auch an den Schuh zusätzlich unterseitig wenigstens ein durch Materialabtrag, insbesondere Fräsen hergestellter Freibereich (4) eingebracht wird.

5. Verfahren nach einem der vorhergehenden Ansprüche, **dadurch gekennzeichnet, dass** der Materialabtrag computergesteuert erfolgt.

6. Verfahren nach einem der vorhergehenden Ansprüche, **dadurch gekennzeichnet, dass** der Materialabtrag entsprechend einer 3D-Messung des Fußes und/oder einer Ganganalyse erfolgt.

7. Verfahren nach einem der vorhergehenden Ansprüche, **dadurch gekennzeichnet, dass** der Materialabtrag entsprechend einer beabsichtigten Kompensation einer diagnostizierten Fehlstellung erfolgt.

8. Einlage (1) für einen Schuh, die eine während der Benutzung zumindest mit der Fußsohle des Benutzers in Kontakt stehende Oberseite (2) und eine während der Benutzung zumindest mit der unteren Innenfläche des Schuhs in Kontakt stehende Unterseite (3) aufweist, und die mit einer auf den Benutzer und insbesondere auch auf den Schuh, abgestimmten Form versehen ist, insbesondere hergestellt nach einem Verfahren der vorhergehenden Ansprüche, ***wobei*** die Einlage (1) personalisiert und einstückig aus einem Vollmaterial durch Materialabtrag, wie z. B. Fräsen, hergestellt ist und zumindest oberseitig eine auf den Benutzer und insbesondere auch auf den Schuh abgestimmte Topografie aufweist, **dadurch gekennzeichnet, dass** *das Vollmaterial einen dreischichtigen Aufbau aufweist, der eine unterseitige* Schicht *mit höherer Härte,* einen *mittleren Bereich geringerer Härte und einen oberen Bereich mit wieder höherer Härte beinhaltet.*

9. Einlage (1) nach Anspruch 8, **dadurch gekennzeichnet, dass** das Vollmaterial ein Thermoplast, insbesondere EVA (Ethylenvinylacetat) ist.

10. Einlage (1) nach Anspruch 8 oder 9, **dadurch gekennzeichnet, dass** sowohl die Oberseite (2) als auch die Unterseite (3) eine zumindest weitgehend standardisierte Topographie aufweisen.

11. Einlage (1) nach Anspruch 10, **dadurch gekennzeichnet, dass** die Unterseite (3) in ihrer standardisierten Topografie zur Anpassung der oberseitigen Topografie während der Benutzung an den Benutzer und insbesondere auch an den Schuh zusätzlich unterseitig wenigstens einen durch Materialabtrag hergestellten, insbesondere ausgefrästen Freibereich (4) aufweist.

## Claims

1. Method of manufacturing an arch support (1) for a shoe, said support having an upper surface (2) contacting, during use, at least the sole of the foot of the user of the arch support (1) and a lower surface (3) contacting, during use, at least the lower interior surface of the shoe and said support being provided with a shape adapted to the user and, in particular, also to the shoe, wherein the arch support (1) is produced in personalized, single-piece form from a solid material by removal of material, e.g. milling, and at least the upper side of the arch support Is provided with a topography adapted to the specific user and, In particular, also to the shoe, ***characterised in that** the solid material has a three-layered structure which comprises a bottom layer of greater hardness, a central area of lower hardness and an upper area again of greater hardness.*

2. Method according to claim 1, **characterised In that** the solid material is a thermoplastic, especially EVA (ethylene vinyl acetate).

3. Method according to any one of the preceding claims, **characterised In that** both the upper surface (2) and the lower surface (3) are provided with an at least largely standardised topography.

4. Method according to claim 3, **characterised in that**, in order to adapt the upper side topography of the arch support (1) during use to the user and, in particular, also to the shoe, at least one free area (4) produced by removal of material, especially milling, is Introduced additionally on the lower side into the standardised topography of the lower surface (3).

5. Method according to any one of the preceding claims, **characterised In that** the removal of material is computer controlled.

6. Method according to any one of the preceding claims, **characterised in that** the removal of material is performed according to 3D measuring of the foot and/or a gait analysis.

7. Method according to any one of the preceding claims, **characterised in that** the removal of material is performed according to an Intended compensation of a diagnosed malallgnment.

8. Arch support (1) for a shoe, said support having an upper surface (2) contacting, during use, at least the sole of the foot of the user and a lower surface (3) contacting, during use, at least the lower interior surface of the shoe and said support being provided with a shape adapted to the user and, in particular, also to the shoe, especially manufactured according to a method of the preceding claims, wherein the arch support (1) is produced in personalized, single-piece form from a solid material by removal of material, e.g. milling, and at least the upper side of the arch support has a topography adapted to the user and, In particular, also to the shoe, ***characterised In that** the solid material has a three-layered structure which comprises a bottom layer of greater hardness, a central area of lower hardness and an upper area again of greater hardness.*

9. Arch support (1) according to claim 8, **characterised In that** the solid material Is a thermoplastic, especially EVA (ethylene vinyl acetate).

10. Arch support (1) according to claim 8 or 9, **characterised In that** both the upper surface (2) and the lower surface (3) have an at least largely standardised topography.

11. Arch support (1) according to claim 10, **characterised In that,** in order to adapt the upper side topography during use to the user and, in particular, also to the shoe, the lower surface (3) In its standardised topography has, additionally on the lower side, at least one free area (4) produced by removal of material, especially milling.

## Revendications

1. Procédé de fabrication d'une semelle intérieure (1) destinée à une chaussure et comprenant une face supérieure (2) qui, au cours de l'utilisation, est au moins en contact avec la plante du pied de l'utilisateur de ladite semelle intérieure (1), et une face inférieure (3) qui, au cours de l'utilisation, est au moins en contact avec la surface interne inférieure de la chaussure, ladite semelle étant dotée d'une forme coordonnée avec l'utilisateur, et notamment aussi avec la chaussure, ladite semelle intérieure (1) étant d'une fabrication personnalisée et d'un seul tenant en un matériau massif, par un enlèvement de matière tel qu'un fraisage, par exemple, et étant pourvue, au moins à la face supérieure, d'une configuration profilée coordonnée avec l'utilisateur effectif, et notamment aussi avec la chaussure, **caractérisé par le fait que** le matériau massif offre une structure à couche triple renfermant une couche inférieure de dureté supérieure, une région médiane de dureté moindre, et une région supérieure de dureté à nouveau supérieure.

2. Procédé selon la revendication 1, **caractérisé par le fait que** le matériau massif est une matière thermoplastique, notamment de l'EVA (copolymère éthylène/acétate de vinyle).

3. Procédé selon l'une des revendications précédentes, **caractérisé par le fait qu'**à la fois la face supérieure (2), et la face inférieure (3), sont munies d'une configuration profilée au moins amplement standardisée.

4. Procédé selon la revendication 3, **caractérisé par le fait qu'**au moins une région dépouillée (4), produite par enlèvement de matière et en particulier par fraisage, est additionnellement ménagée à la face inférieure dans la configuration profilée standardisée de ladite face inférieure (3), de telle sorte que la configuration profilée de la face supérieure de la semelle intérieure (1) soit adaptée, au cours de l'utilisation, à l'utilisateur et notamment aussi à la chaussure.

5. Procédé selon l'une des revendications précédentes, **caractérisé par le fait que** l'enlèvement de matière est effectué avec commande par ordinateur.

6. Procédé selon l'une des revendications précédentes, **caractérisé par le fait que** l'enlèvement de matière est effectué en concordance avec une mesure du pied en 3D, et/ou avec une analyse de l'allure déambulatoire.

7. Procédé selon l'une des revendications précédentes, **caractérisé par le fait que** l'enlèvement de matière est effectué en concordance avec une compensation ciblée d'une posture défectueuse diagnostiquée.

8. Semelle intérieure (1) destinée à une chaussure et comprenant une face supérieure (2) qui, au cours de l'utilisation, est au moins en contact avec la plante du pied de l'utilisateur, et une face inférieure (3) qui, au cours de l'utilisation, est au moins en contact avec la surface interne inférieure de la chaussure, ladite semelle étant dotée d'une forme coordonnée avec l'utilisateur, et notamment aussi avec la chaussure, fabriquée en particulier selon un procédé conforme aux revendications précédentes, ladite semelle intérieure (1) étant d'une fabrication personnalisée et d'un seul tenant en un matériau massif, par un enlèvement de matière tel qu'un fraisage, par exemple, et étant pourvue, au moins à la face supérieure, d'une configuration profilée coordonnée avec l'utilisateur, et notamment aussi avec la chaussure, **caractérisée par le fait que** le matériau massif offre une structure à couche triple renfermant une couche inférieure de dureté supérieure, une région médiane de dureté moindre, et une région supérieure de dureté à nouveau supérieure.

9. Semelle intérieure (1) selon la revendication 8, **caractérisée par le fait que** le matériau massif est une matière thermoplastique, notamment de l'EVA (copolymère éthylène/acétate de vinyle).

10. Semelle intérieure (1) selon la revendication 8 ou 9, **caractérisée par le fait qu'**à la fois la face supérieure (2), et la face inférieure (3), sont munies d'une configuration profilée au moins amplement standardisée.

11. Semelle intérieure (1) selon la revendication 10, **caractérisée par le fait que** la face inférieure (3) comporte, dans sa configuration profilée standardisée, au moins une région dépouillée (4) produite par enlèvement de matière et notamment rognée par fraisage, additionnellement ménagée à ladite face inférieure, de telle sorte que la configuration profilée de la face supérieure soit adaptée, au cours de l'utilisation, à l'utilisateur et notamment aussi à la chaussure.
